(19) Europäisches Patentamt
European Patent Office
Office européen des brevets

(11) EP 2 002 851 A2

(12) EUROPEAN PATENT APPLICATION
published in accordance with Art. 153(4) EPC

(43) Date of publication:
17.12.2008 Bulletin 2008/51

(51) Int Cl.:
A61L 27/00 (2006.01)     C08J 9/28 (2006.01)

(21) Application number: 07739214.0

(22) Date of filing: 20.03.2007

(86) International application number:
PCT/JP2007/055771

(87) International publication number:
WO 2007/111205 (04.10.2007 Gazette 2007/40)

(84) Designated Contracting States:
DE FR GB IT NL

(30) Priority: 20.03.2006 JP 2006076177
20.03.2007 JP 2007073714

(71) Applicant: JMS Co., Ltd.
Hiroshima-shi, Hiroshima 730-8652 (JP)

(72) Inventors:
• SAJIKI, Toshinobu
Hiroshima-shi, Hiroshima 730-8652 (JP)
• HANAKI, Naoyuki
Hiroshima-shi, Hiroshima 730-8652 (JP)
• IDE, Junichi
Hiroshima-shi, Hiroshima 730-8652 (JP)
• HIRAYAMA, Fumiko
Hiroshima-shi, Hiroshima 730-8652 (JP)

(74) Representative: Röthinger, Rainer
c/o Wuesthoff & Wuesthoff
Patent- und Rechtsanwälte
Schweigerstrasse 2
D-81541 München (DE)

(54) **POROUS BIOABSORBABLE MATERIAL AND METHOD OF PRODUCING THE SAME**

(57)     [PROBLEMS]

To provide a thin film-shaped porous bioabsorbable material that is a dense structure in which the pore size average is small and the pore size is uniform and which further has a large maximum stress, in particular, a porous bioabsorbable material extremely useful as an adhesion preventing material.

[MEANS FOR SOLVING PROBLEMS]

A porous bioabsorbable material **characterized by** being formed of a porosified bioabsorbable polymer which is obtained in a manner that a bioabsorbable polymer is gelled with a good solvent and a poor solvent, compatible with each other, for the bioabsorbable polymer and the gelled bioabsorbable polymer is subjected to freeze-drying to be porosified; and a method of producing the porous bioabsorbable material, the method being **characterized in that** a bioabsorbable polymer is gelled with a mixed solvent composed of a good solvent and a poor solvent, compatible with each other, for the bioabsorbable polymer and the gelled bioabsorbable polymer is subjected to freeze-drying to be porosified.

[Fig.1]

EP 2 002 851 A2

## Description

### Technical Field

The present invention relates to a porous body, in particular, a porous bioabsorbable material useful in the medical field centering on the tissue engineering and the regenerative medical engineering and a method of producing the porous bioabsorbable material.

### Background Art

Bioabsorbable materials used in intravital implant materials are mainly used in regenerative therapy scaffold materials and in adhesion preventing materials. For the former regenerative therapy scaffold materials, porous bodies are preferably utilized for the purpose of proliferating cells in the interior of the regenerative therapy scaffold materials. When a porous body is utilized, cells are seeded in the pores of the porous body to be proliferated therein, the porous body is transplanted in a living organism, and herewith the tissue regeneration occurs and at the same time, the bioabsorbable material serving as the scaffold is gradually decomposed and absorbed within the living organism. Accordingly, the scaffold used for the proliferation of the cells can be transplanted as it is, in a living organism together with the proliferated cells. Additionally, when a bioabsorbable polymer is used as the porous regenerative therapy scaffold material, desired is a porous body having a relatively large pore size in order to allow the cells to penetrate into the porous body.

As the method of producing the porous body to be used in the porous regenerative therapy scaffold material, the freeze dry methods as described, for example, in Patent Documents (1) to (4) listed below are known. Further, the present inventors have proposed a method of producing a bioabsorbable material in the following manner: a freeze dry treatment is applied to a mixed solution containing a polymer including a copolymer of lactide and caprolactone, a solvent (poor solvent) relatively lower in the solubility to the polymer and a solvent (good solvent) relatively higher in the solubility to the polymer and compatible with the solvent lower in solubility; in this way, the bioabsorbable material is produced by porosifying a bioabsorbable polymer which solves the above-described problems of the conventional techniques and permits controlling the pore size over a wide range from a small pore size to a large pore size, and the production process of which is simple (Patent Document 5).

Patent Document 1: Japanese Patent Laid-Open No. 10-234844

Patent Document 2: Japanese Patent Laid-Open No. 2001-49018

Patent Document 3: National Publication of International Patent Application No. 2002-541925

Patent Document 4: Japanese Patent Laid-Open No. 02-265935

Patent Document 5: Japanese Patent Application No. 2005-80059

### Disclosure of the Invention

### Problems to be Solved by the Invention

A porous bioabsorbable material, in particular, a porous bioabsorbable material used in an adhesion preventing material has a porous structure to be used for supplying nutrient components and the like to a living tissue to be brought into contact with the concerned material. However, at the same time, the pore size of the porous structure is required to be relatively small for the purpose of preventing the tissue from the adhesion due to the penetration of the cells into the pores. Further, an adhesion preventing material is usually used in a form of a thin film, and accordingly, a bioabsorbable material which has a large stress in spite of having a form of a porous thin film has been desired.

However, currently generally adopted methods of producing thin films of porous bioabsorbable materials have never been able to produce porous thin films to meet the above-described requirements. For example, a thin film of a porous bioabsorbable material of 300 $\mu$m or less is too weak in the strength thereof, and accordingly causes problems such that suturing cannot be conducted, breakage tends to occur, holes are created, and releasing from a mold is difficult. For the purpose of solving these problems, it is a possible remedy to increase the film thickness of the adhesion preventing material. However, this remedy causes a problem that it is difficult to insert the adhesion preventing material into and properly dispose the adhesion preventing material in the treatment spot needing the adhesion preventing material.

Accordingly, it has been difficult to acquire a porous bioabsorbable material which is a porous thin film and at the same time has a large strength, in particular, a strength required for an adhesion preventing material.

The present invention resides in the provision of a porous bioabsorbable material, in particular, a porous bioabsorbable material which has relatively small pores, in a uniform manner, to prevent the penetration of cells and at the same time to be suitable for the permeation of the substances such as nutritional support substances, and which is a porous body, at the same time has a large maximum stress and thus is useful as an adhesion preventing material, and in the provision of a method of producing the porous bioabsorbable material.

**Means for Solving the Problems**

The present inventors were able to solve the above-described technical problems by providing a porous bioabsorbable material characterized in that the bioabsorbable material has a maximum stress of 3 to 23 (MPa), a porosity of 0.1 to 82(%) and a pore size diameter (average) (hereinafter also referred to as a pore size average) of 9 to 34 ($\mu$m), and by providing a method of producing the porous bioabsorbable material.

The porous bioabsorbable material of the present invention can be produced by a step of preparing a gelled product by using a bioabsorbable polymer, a good solvent for the bioabsorbable polymer and a poor solvent for the bioabsorbable polymer, these two solvents being compatible with each other, a step of subjecting the gelled product to a freeze treatment and a step of drying under reduced pressure the freeze-treated substance. It is to be noted that the good solvent means the solvent having a relatively larger solubility to the bioabsorbable polymer and the poor solvent means the solvent having a relatively smaller solubility to the bioabsorbable polymer.

The step of preparing the gelled product is conducted by phase-separating, into the solvent phase and the gel phase, the mixture composed of at least the bioabsorbable material, the good solvent and the poor solvent through controlling the mixing amount of the poor solvent in the mixture; the mixing amount of the poor solvent required for the phase separation is varied depending on the various factors such as the composition or composition ratio and molecular weights of the monomer components making up the bioabsorbable material, the combination of and the ratio between the bioabsorbable material, the good solvent and the poor solvent, and the ambient temperature.

Hereinafter, the present invention is described in detail on the basis of embodiments.

1. Bioabsorbable polymer

As the bioabsorbable polymer in the present invention, for example, a copolymer of lactide and caprolactone may be quoted. The copolymer may be either a random polymer or a block polymer. The molecular weight (weight average molecular weight) of the copolymer is not particularly limited, but is, for example, 5,000 to 2,000,000, preferably 10,000 to 1,500,000 and more preferably 100,000 to 1,000,000. Additionally, the molecular ratio of lactide to caprolactone falls, for example, in a range of 90:10 to 10:90, preferably in a range of 85:15 to 20:80 and more preferably in a range of 80:20 to 40:60.

The method of polymerizing the copolymer of lactide and caprolactone is not particularly limited, and a heretofore known method may be adopted as the concerned method. For example, lactide and caprolactone as the starting materials may be copolymerized by ring-opening polymerization, or lactide (cyclic dimmer of lactic acid) may be synthesized from lactic acid and then the lactide may be copolymerized with caprolactone.

As the lactide, L-lactide, D-lactide and a mixture (D,L-lactide) thereof can be used, and as lactic acid, L-lactic acid, D-lactic acid and a mixture (D,L-lactic acid) thereof can be used. When lactic acid is used as the starting material as described above, the amount of the monomer, lactic acid is converted into the amount of the dimmer, lactide, and the molar ratio between the thus converted amount of lactide and the amount of caprolactone preferably falls within the above-described ranges. Additionally, as the lactone, for example, $\epsilon$-caprolactone, $\gamma$-butyrolactone and $\delta$-valerolactone are quoted, and preferable among these is $\epsilon$-caprolactone.

In the above description, a specific description has been made by taking as an example the copolymer of lactide and caprolactone as the bioabsorbable polymer of the present invention to be the target of porosification; however, even other bioabsorbable polymers are also included in the porosified bioabsorbable polymer of the present invention as long as the other bioabsorbable polymers can be gelled by the solvent composed of a good solvent and a poor solvent for the polymers. Such bioabsorbable polymers may also be those polymers which contain, as the constituent components, copolymerization components constituting other bioabsorbable polymers other than lactide and caprolactone. Examples of such copolymerization components may include those copolymerization components derived from glycolic acid, tri-methylene carbonate, $\beta$-hydorxybutyric acid, proteins and sugars.

2. Gelled product

The gelled product of the bioabsorbable polymer used in the present invention is prepared by separating the bioabsorbable polymer phase-separated as a gelled state by mixing the poor solvent in an amount required for gelling the bioabsorbable polymer in the mixture containing the bioabsorbable polymer, and the good solvent and the poor solvent for the bioabsorbable polymer compatible with each other. The amount of the bioabsorbable polymer in the mixture is not particularly limited, but is usually 0.1 to 24% by mass, preferably 2 to 8% by mass and more preferably 3 to 5% by mass.

3. Solvents

The types of the good solvent and the poor solvent compatible with each other are determined, for example, according to the type of the bioabsorbable polymer used. As the poor solvent necessary to prepare the copolymer of lactide and caprolactone, water, ethanol, tertiary-butyl alcohol (tBuOH) and the like can be used; as the good solvent, organic solvents exhibiting compatibility with the poor solvent such as 1,4-dioxane and dimethyl carbonate can be used; in particular, a combination of water as the poor solvent and 1,4-dioxane as the good solvent is preferable.

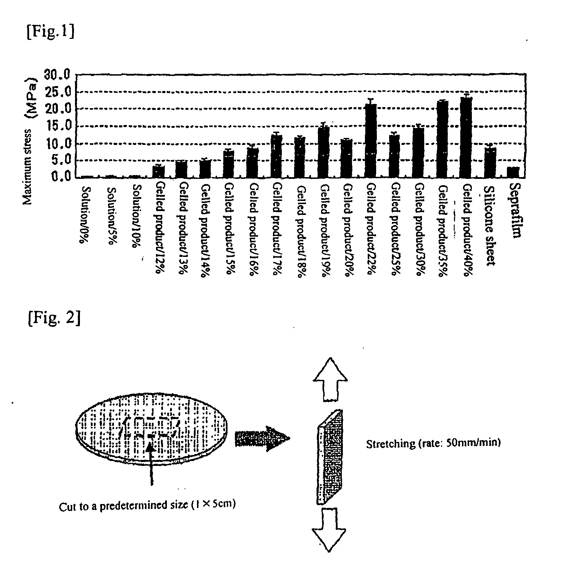

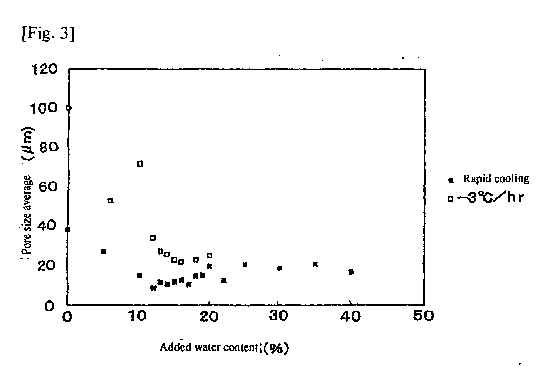

In the mixture containing the bioabsorbable polymer, the poor solvent and the good solvent, the mixing amount of the poor solvent required for gelling the bioabsorbable polymer can be appropriately determined according to the types and the like of the bioabsorbable polymer, the poor solvent and the good solvent constituting the mixture; when the mixing amount of the poor solvent falls short of the amount required for converting the mixture into a gelled state, no gelled state is formed; on the contrary, when the mixing amount of the poor solvent excessively exceeds the amount required for forming a gelled state, the bioabsorbable polymer is excessively coagulated and is partially converted into a state of film, and hence no sufficient porosification can be effected even by conducting freeze drying. Accordingly, the amount of the poor solvent required for allowing the mixture to form the gelled state is appropriately determined, in a specified manner, according to the adopted mixture containing the bioabsorbable polymer, the poor solvent and the good solvent. Additionally, by varying the mixing amount of the poor solvent within a range permitting preparing the gelled product, the porosity of the porous bioabsorbable polymer can be controlled as shown in Table 3, the maximum stress of the porous bioabsorbable polymer can be controlled as shown in Table 4 and Figure 1, or the pore size average of the porous bioabsorbable polymer can be controlled as shown in Table 2, Tables 6 to 8 and Figure 3.

Freezing step

For freezing of the gelled product, a heretofore known freezing step and a heretofore known freezing apparatus can be used. Additionally, the freezing temperature of the gelled product is not particularly limited as long as the freezing temperature is equal to or lower than the eutectic point at which the gelled product is completely frozen; however, in the case of the gelled product in which the bioabsorbable polymer is the copolymer of lactide and caprolactone, the freezing point falls preferably in a range of -3°C or lower and more preferably in a range of -10°C or lower. Additionally, by varying the cooling rate of the gelled product, the pore size of the obtained porous bioabsorbable material is varied, and hence by selecting the cooling rate of the gelled product, the pore size of the porous bioabsorbable material can be controlled.

**Brief Description of Drawings**

[Figure 1]
Figure 1 is a graph showing the maximum stresses of the porous bioabsorbable materials of Example 1 and comparative examples.
[Figure 2]
Figure 2 is a view illustrating a method of measuring the maximum stress of a porous bioabsorbable material.
[Figure 3]
Figure 3 is a graph showing the pore size averages of porous bioabsorbable materials.
[Figure 4]

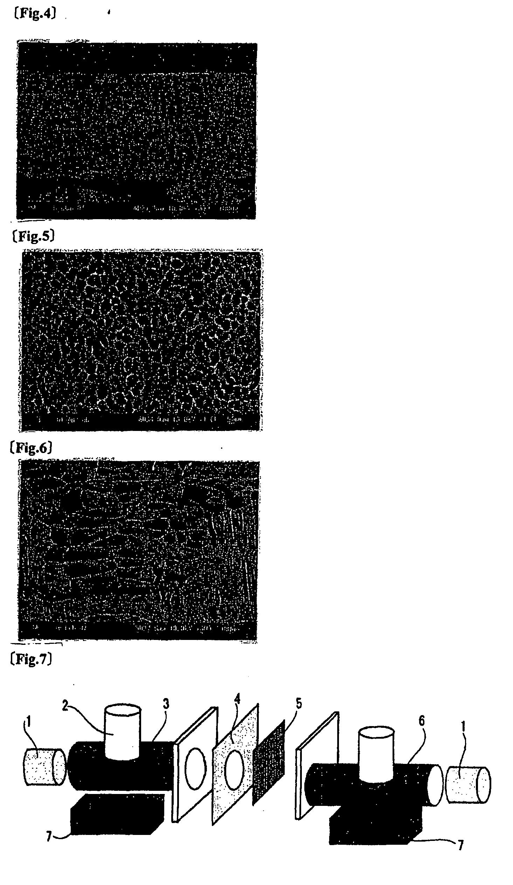

Figure 4 is a sectional micrograph of a porous body E obtained by using an electron microscope (SEM) at a magnification factor of 300.
[Figure 5]
Figure 5 is a surface micrograph of the porous body E obtained by using an electron microscope (SEM) at a magnification factor of 1000.
[Figure 6]
Figure 6 is a sectional micrograph of a porous body A with an added water content of 0%, obtained by using an electron microscope (SEM) at a magnification factor of 100.
[Figure 7]

Figure 7 is a view illustrating the glucose permeability tester used in Example 3.
[Figure 8]

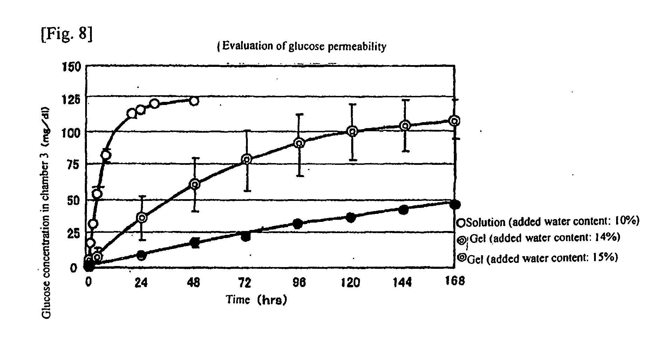

Figure 8 is a graph showing the results of the glucose permeability test in Example 3.

**Description of Reference Numerals**

| 1 | Silicone stopper |
| 2 | Sampling port |
| 3 | Chamber (glucose side) |
| 4 | Silicone film |
| 5 | Specimen film made of a porous body thin film prepared from Sample D, E or A. |
| 6 | Chamber (RO water side) |
| 7 | Stirrer |

**Best Mode of Carrying Out the Invention**

By varying the water content of a mixed solution, a bioabsorbable polymer is porosified to prepare porous bioabsorbable materials.

Example 1

Preparation of porous lactide-caprolactone copolymers by rapid cooling

A lactide-caprolactone copolymer (hereinafter, also referred to as LA/CL copolymer) having a composition ratio (molar ratio) of L-lactide to $\varepsilon$-caprolactone of 75:25, 1,4-dioxane and water were mixed together, and thus 17 types of mixed solutions A to R listed in Table 1 presented below were prepared each in an amount of 12 g. Among these samples, Samples D to R were gelled, but Samples A to C remained in a solution state.

In Table 1 presented below, the compositions of Samples A to R are shown.

[Table 1]

| Sample | Added water content (%) | P (LA/CL) | Dioxane | Water |
|--------|------------------------|-----------|---------|-------|
| A | 0 | 4.00 | 96.00 | 0 |
| B | 5 | 3.80 | 91.20 | 5 |
| C | 10 | 3.60 | 86.40 | 10 |
| D | 12 | 3.52 | 84.48 | 12 |
| E | 13 | 3.48 | 83.52 | 13 |
| F | 14 | 3.44 | 82.56 | 14 |
| G | 15 | 3.40 | 81.60 | 15 |
| H | 16 | 3.36 | 80.64 | 16 |
| I | 17 | 3.32 | 79.68 | 17 |
| J | 18 | 3.28 | 78.72 | 18 |
| K | 19 | 3.24 | 77.76 | 19 |
| L | 20 | 3.20 | 76.80 | 20 |
| M | 22 | 3.12 | 74.88 | 22 |
| N | 25 | 3.00 | 72.00 | 25 |
| O | 30 | 2.80 | 67.20 | 30 |
| P | 35 | 2.60 | 62.40 | 35 |
| R | 40 | 2.40 | 57.60 | 40 |

In the present invention inclusive of the description in the above table, the "added water content" is represented in terms of percent by weight.

Samples D to R were phase-separated and thus the gelled products of the L-lactide-ε-caprolactone copolymer were separated. The thus separated gelled products were each supplied to a stainless steel Petri dish and were each formed in a thickness of 0.5 mm. Additionally, Sample A in a solution state was also supplied to a stainless steel Petri dish. The Petri dishes containing the gelled products or the solutions were placed on a cooling rack cooled at -50°C of a freeze-dryer, for example, TF 5-85 ATANCS (trade name, manufactured by Takara Seisakusho) and subjected to a rapid freezing over a period of approximately 1 hour. Thereafter, the temperature inside the freeze-dryer was increased from - 50°C to 25°C under a reduced pressure over a period of 12 hours and thus porous bodies were prepared. The pore size averages (μm) of these porous bodies are shown in Table 2 presented below and in Figure 3, and the porosities of these porous bodies are shown in Table 3 presented below.

In Table 2 presented below, the pore size averages (μm) of the porous bodies A to R are shown. In Table 2 presented below, when the added water content is 10% or less, the pore size average is large, and the pore size average was largely varied depending on the added water content. Additionally, as can be seen from Table 2, when the added water content is 10% or less, the standard deviation of the pore size average is large and the pore sizes of the obtained porous bodies undergo variation. On the other hand, as can be seen from Table 2, when the added water content is 12% or more, the pore size average is small and the standard deviation of the pore size average is very small. Thus, as can be seen, according to the present invention, porous bodies relatively small in pore size and relatively uniform in pore size are obtained.

[Table 2]

| Sample | Added water content (%) | Pore size average (μm) | Standard deviation |
|---|---|---|---|
| A | 0 | 38 | 6.1 |
| B | 5 | 27 | 5.4 |
| C | 10 | 15 | 0.8 |
| D | 12 | 9 | 0.0 |
| E | 13 | 12 | 1.4 |
| F | 14 | 11 | 0.6 |
| G | 15 | 12 | 1.5 |
| H | 16 | 13 | 1.3 |
| I | 17 | 11 | |
| J | 18 | 15 | |
| K | 19 | 15 | |
| L | 20 | 20 | |
| M | 22 | 13 | |
| N | 25 | 20 | |
| O | 30 | 19 | |
| P | 35 | 21 | |
| R | 40 | 17 | |

A sectional micrograph of the porous body E at a magnification factor of 300 based on an electron microscope (SEM) was taken and is shown in Figure 4. Additionally, a surface micrograph of the same porous body at a magnification factor of 1000 based on an electron microscope (SEM) was taken and is shown in Figure 5. As can be seen from these figures, the porous bodies obtained from the gelled products of present Example are approximately uniform in pore size diameter.

On the other hand, the mixture (Sample A) having a mixing ratio between the LA/CL copolymer, 1,4-dioxane and water of 4:96:0 (% by weight), in a sate of a solution containing no water mixed therein, was subjected to rapid freeze drying in the same manner as in Example 1 to prepare a porous body; and a sectional micrograph of the thus obtained porous body was taken with an electron microscope (SEM) at a magnification factor of 100 and is shown in Figure 6. As shown in Figure 6, the pores of the porous body have locally different orientations (for example, an orientation in the transverse direction or in the longitudinal direction), and the pore size diameters fall in a wide range from a remarkably large diameter to a remarkably small one.

In Table 3 presented below, the porosities (%) of the porous bodies A to R are shown.

[Table 3]

| Sample | Added water content | Porosity (%) |
|--------|---------------------|--------------|
| A | 0 | 92 |
| B | 5 | 91 |
| C | 10 | 92 |
| D | 12 | 82 |
| E | 13 | 74 |
| F | 14 | 69 |
| G | 15 | 67 |
| H | 16 | 61 |
| I | 17 | 54 |
| J | 18 | 43 |
| K | 19 | 45 |
| L | 20 | 37 |
| M | 22 | 20 |
| N | 25 | 26 |
| O | 30 | 18 |
| P | 35 | 12 |
| R | 40 | 0.1 |

In Table 4 presented below, the maximum stresses (MPa) of the porous bodies A to R are shown.

[Table 4]

| Sample | Added water content (%) | Average maximum stress (Mpa) |
|--------|-------------------------|------------------------------|
| A | 0 | 0.4 |
| B | 5 | 0.5 |
| C | 10 | 0.6 |
| D | 12 | 3.4 |
| E | 13 | 4.7 |
| F | 14 | 5.2 |
| G | 15 | 7.7 |
| H | 16 | 8.5 |
| I | 17 | 13 |
| J | 18 | 12 |
| K | 19 | 15 |
| L | 20 | 11 |
| M | 22 | 21 |

(continued)

| Sample | Added water content (%) | Average maximum stress (Mpa) |
|--------|--------------------------|-------------------------------|
| N | 25 | 12 |
| O | 30 | 15 |
| P | 35 | 22 |
| R | 40 | 23 |

Example 2

Above-described Samples A, C to H, J and L, and in addition to these samples, Sample S in a solution state, shown in Table 5 presented below were prepared according to the preparation method adopted in Example 1. These Samples were converted into molded bodies in the same manner as in Example 1, and from these molded bodies, porous bodies were prepared by adopting a cooling rate of -3°C/hr in the same method as the method of preparing the porous bodies in Example 1. Further, Samples E, F and G were converted into molded bodies in the same manner as in Example 1, and from these molded bodies, porous bodies were prepared by adopting cooling rates of -5°C/hr and -10°C/hr in the same method as the method of preparing the porous bodies in Example 1. The pore size averages ($\mu$m) of these prepared porous bodies are shown in Tables 7 and 8.

In Table 5 presented below, the composition of Sample S is shown.

[Table 5]

| Sample | Added water content (%) | P(LA/CL) | Dioxane | Water |
|--------|--------------------------|----------|---------|-------|
| S | 6 | 3.76 | 90.24 | 6 |

In Table 6 presented below, the pore size averages of the porous bodies prepared at a cooling rate of -3°C/hr are shown.

[Table 6]

| Sample | Added water content | Pore size average ($\mu$m) |
|--------|----------------------|-----------------------------|
| A | 0 | 100 |
| S | 6 | 53 |
| C | 10 | 72 |
| D | 12 | 34 |
| E | 13 | 27 |
| F | 14 | 26 |
| G | 15 | 23 |
| H | 16 | 22 |
| J | 18 | 23 |
| L | 20 | 25 |

In the above table, when the added water content is 10% or less, the pore size average is large and the pore size average is largely varied depending on the added water content.

In Table 7 presented below, the pore size averages of the porous bodies prepared at a cooling rate of -5°C/hr are shown.

[Table 7]

| Sample | Added water content (%) | Pore size average ($\mu$m) |
|--------|--------------------------|-----------------------------|
| E | 13 | 23 |

(continued)

| Sample | Added water content (%) | Pore size average ($\mu$m) |
|---|---|---|
| F | 14 | 21 |
| G | 15 | 21 |

In Table 8 presented below, the pore size averages of the porous bodies prepared at a cooling rate of -10°C/hr are shown.

[Table 8]

| Sample | Added water content (%) | Pore size average ($\mu$m) |
|---|---|---|
| E | 13 | 20 |
| F | 14 | 18 |
| G | 15 | 17 |

As can be seen from the results of Examples 1 and 2, when the gelled products of the lactide-caprolactone copolymer are freeze-dried, in the case where slow cooling is conducted as in Example 2, as compared to the case where rapid cooling is conducted as in Example 1, the pore size average becomes somewhat larger, but is small in the case of slow cooling as well as in the case of rapid cooling, and the magnitudes of the pore size averages are also uniform. On the other hand, in the case where the freeze drying of the solution of the lactide-caprolactone copolymer is conducted by slow cooling, as can be seen from Table 6 presented above and Figure 3 and Figure 6, the pore size average becomes large and the pore size average is largely varied depending on the mixing amount of water as the poor solvent.

The pore size average of each of the porous bodies prepared in Examples 1 and 2 was measured in the following manner. An obtained disc-shaped porous body thin film was cut to expose a section thereof. The section of the cut porous body sample was observed with an electron microscope, the magnification of the electron microscope was set so as to permit identifying pore sizes for approximately 30 to 100 pores in a field of view, and then a SEM micrograph was photographed. In a thus obtained SEM image, ten pores having relatively large pore sizes and having pore sizes high in rate of occurrence were selected, and then the ten pores were analyzed with an image analysis software (NIH image) to derive the pore size average.

Maximum stresses of porous bodies

The maximum stress of each of the porous bodies prepared in Example 1 was measured in the following manner. As shown in Figure 2, an obtained disc-shaped porous body sample was cut to a 1 x 5 cm rectangular strip to prepare a specimen. The maximum stress of the specimen was measured by using a tensile test machine (trade name: Autograph, manufactured by Shimadzu Corp.) and by stretching the specimen with a separation between the chucks of 20 mm at the time of testing and at a stretching rate of 50 mm/min. The measurement results are shown in Figure 1. Additionally, as a comparative example, the maximum stress of a commercially available synthetic absorbable adhesion preventing material (trade name: Seprafilm (trade mark), manufactured by Genzyme Japan K. K.) was measured, and the result obtained is shown in Figure 1. As can be seen from the results shown in Figure 1, the porous bodies prepared from the gelled products (Samples D to R) of the lactide-caprolactone copolymer are larger in the maximum stress as compared to the porous bodies prepared from the solutions (Sample A, B and C) of the lactide-caprolactone copolymer, and each have a large maximum stress approximately equal to or larger than the maximum stress of a silicone sheet that is a nonporous body. Additionally, the porous bodies according to the present invention each have a maximum stress larger than the maximum stress of the commercially available synthetic absorbable adhesion preventing material that was used as a comparative example.

Porosities of porous bodies

The porosity of each of the porous bodies prepared in Example 1 was measured in the following manner. A disc-shaped porous body was cut to a 1.5 x 1.5 cm square shape to prepare a specimen, and the weight of the specimen was measured. Then the film thickness of the specimen was measured with a digital microscope (manufactured by Keyence Corp.). From the obtained weight and film thickness of the specimen, the density ($\rho$) of the porous body sample was derived. Additionally, the density ($\rho_0$) of a film prepared from the lactide-caprolactone copolymer having the same composition as that of the concerned porous body sample was measured in the same manner as described above. Then, the porosity (%) (p) was derived on the basis of the following formula:

$$p = 1 - \rho/\rho_0$$

As can be seen from the test results of the porosities shown in Table 3 presented above, the porous bodies prepared from the gelled products of the lactide-caprolactone copolymer exhibit a remarkable decrease of the porosity with the increase of the mixing amount of water as the poor solvent, in contrast to the porous bodies prepared from the solutions of the same lactide-caprolactone copolymer.

As can be seen from the test results of Examples 1 and 2, the porous bodies prepared from the gelled products of the lactide-caprolactone copolymer, as compared to the porous bodies prepared from the solutions of the same lactide-caprolactone copolymer, (1) are extremely large in maximum stress, (2) are dense structures remarkably small in porosity, and (3) provide the porous bodies in which irrespective as to whether the cooling method in the freeze-drying step adopts rapid cooling or slow cooling, the variation of the pore size average is small, the pore size is uniform, and the pore size average is small; further, by varying the mixing amount of water as the poor solvent in the gel preparation step, irrespective as to whether the cooling method in the freeze-drying step adopts rapid cooling or slow cooling, the pore size average itself can be varied while being maintained is the above-described characteristic that the pore size is small and uniform and the pore size average is small. Further, as can be seen from the test results of Example 1, the porous bodies of Samples D to R, prepared from the gelled products of the lactide-caprolactone copolymer offer the bioabsorbable materials that yield the maximum stress of 3.4 to 23.1 (MPa), the porosity (%) of 0.1 to 82% and the pore size average of 9 to 21 ($\mu$m).

Example 3

The thin films of Sample E (added water content: 14%, film thickness: 150 $\mu$m) and Sample G (added water content: 15%, film thickness: 170 $\mu$m) obtained by the preparation method in Example 1 were subjected to a test of the glucose permeability thereof by using a permeability tester shown in Figure 7 in the following manner. Additionally, Sample C (added water content: 10%, film thickness: 100 $\mu$m) as a comparative example was examined in the same manner.

Two chambers (3, 6) each having a volume of 14 mm in diameter x 80 mm in length were connected (secured with screws) liquid-tight to each other through the intermediary of an evaluation sample 5 and a silicone film 4 disposed therebetween. Next, in the chamber 3, 10 mL of a glucose solution having a concentration of 250 mg/dL was filled, and in the chamber 6, 10 mL of RO water was filled. Next, the interior of each of the chambers 3 and 6 was moderately stirred with a stirrer 7, and 20 $\mu$L of the glucose solution was sampled from each of the sampling ports every predetermined time interval to be used as a test specimen. Each of the test specimens was subjected to an analysis with a glucose determination kit (trade name: Glucose CII-Test Wako, manufactured by Wako Pure Chemical Industries, Ltd.) and the glucose concentration in each of the chambers 3 and 6 was determined from the absorbance. The results thus obtained are shown in Figure 8.

As can be seen from the results shown in Figure 8, Sample F (gel, added water content: 14%) and Sample G (gel, added water content: 15%) each have a glucose permeability although the glucose permeability is smaller than that of Sample A (solution, added water content: 10%). A comparison between Sample F and Sample G shows that these Samples are different in the glucose permeability from each other. In this way, the bioabsorbable porous bodies prepared from gels, obtained according to the present invention, each have a glucose permeability, and at the same time, allow to control the glucose permeating abilities thereof by varying the added water content in the preparation process thereof.

Example 4

Preparation of adhesion preventing materials

By using the preparation method of Example 1, the thin films, of 50 to 600 $\mu$m in film thickness, preferably 50 to 300 $\mu$m in film thickness, of the porous lactide-caprolactone copolymer were formed in the stainless steel Petri dishes from the gelled products of the lactide-caprolactone copolymer, of Samples D to R, and then the thin films were taken out of the stainless steel Petri dishes. At the time of this taking-out, no cracks occur in the thin films because the porous lactide-caprolactone copolymer forming the thin films is large in the maximum stress. Additionally, as described above, the thin films of the porous lactide-caprolactone copolymer are dense structures in which the pores of the thin films are relatively small and have uniform size averages, and each of which has a large maximum stress. Accordingly, the porous bioabsorbable material of the present invention is, as described above, of a porous thin film shape, and at the same time, has a large stress; the pores thereof are relatively small so as to be suitable for preventing the penetration of cells, additionally the pore size is uniform and the pore is provided with a glucose permeability, and hence the porous bioabsorbable material is suitable for the permeation of the substances such as nutritional support substances; accordingly, the porous bioabsorbable material of the present invention is extremely useful as the adhesion preventing material such as a tendon adhesion preventing material.

**Industrial Applicability**

According to the method of producing the porous bioabsorbable material of the present invention, as shown in Figure 4, irrespective as to whether the cooling method of the freeze-drying step adopts rapid cooling or slow cooling (-3, -5 and -10°C/hr), the pore size is uniform and the uniform pore size is relatively small. Additionally, by varying the mixing amount of the poor solvent in the stage of the preparation of the gelled product, the porosity, the maximum stress or the pore size average of the porous bioabsorbable polymer can be controlled. Additionally, the porous bioabsorbable material prepared by means of the method of producing a porous bioabsorbable material of the present invention is a dense structure in which the pore size average is small and the pore size is uniform, and which has a large maximum stress. Accordingly, the porous bioabsorbable material is of a porous thin film shape, and at the same time, has a large maximum stress; the pores thereof are relatively small so as to be suitable for preventing the penetration of cells, additionally the pore size is uniform and the pore is provided with a glucose permeability, and hence the porous bioabsorbable material is suitable for the permeation of the substances such as nutritional support substances; accordingly, the porous bioabsorbable material of the present invention is extremely useful as a thin film-shaped porous bioabsorbable material, in particular, an adhesion preventing material.

**Claims**

1.  A porous bioabsorbable material **characterized in that** a bioabsorbable material has a maximum stress of 3 to 23 (MPa), a porosity of 0.1 to 82(%) and a pore size average of 9 to 34 ($\mu$m).

2.  The porous bioabsorbable material according to claim 1, wherein a bioabsorbable polymer is a copolymer of lactide and caprolactone.

3.  The porous bioabsorbable material according to claim 1 or 2, wherein the porous bioabsorbable material is a thin film of 50 to 600 $\mu$m in thickness.

4.  The porous bioabsorbable material according to claim 3, wherein the porous bioabsorbable material is a tendon adhesion preventing material, as a thin film, of 50 to 500 $\mu$m in thickness.

5.  The porous bioabsorbable material according to any one of claims 1 to 4, wherein the bioabsorbable polymer is gelled with a good solvent and a poor solvent, compatible with each other, for the bioabsorbable polymer, and the gelled bioabsorbable polymer is subjected to freeze-drying to produce the porous bioabsorbable material.

6.  A method of producing the porous bioabsorbable material according to any one of claims 1 to 5, **characterized in that** the bioabsorbable polymer is gelled with a mixed solvent composed of a good solvent and a poor solvent, compatible with each other, for the bioabsorbable polymer, and the gelled product is subjected to freeze-drying to be porosified.

7.  The method of producing a porous bioabsorbable material according to claim 6, wherein dioxane and water are combined as the good solvent and the poor solvent, respectively.

8.  The method of producing a porous bioabsorbable material according to claim 6 or 7, wherein the mixing amount of the poor solvent is 12 to 40% by weight.

9.  The method of producing a porous bioabsorbable material according to any one of claims 6 to 8, wherein the pore size of the porous bioabsorbable polymer is controlled by varying the cooling rate of the gelled product.

10. The method of producing a porous bioabsorbable material according to any one of claims 6 to 9, wherein the porosity of the porous bioabsorbable polymer is controlled by varying the mixing amount of the poor solvent in the preparation stage of the gelled product.

[Fig.1]

[Fig. 2]

Cut to a predetermined size (1 × 5cm)

Stretching (rate: 50mm/min)

[Fig. 3]

[Fig.4]

[Fig.5]

[Fig.6]

[Fig.7]

[Fig. 8]

( Evaluation of glucose permeability

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10234844 A
- JP 2001049018 A
- JP 2002541925 A
- JP 2265935 A
- JP 2005080059 A